(19) Europäisches Patentamt
European Patent Office
Office européen des brevets

(11) EP 0 873 290 B1

(12) EUROPÄISCHE PATENTSCHRIFT

(45) Veröffentlichungstag und Bekanntmachung des Hinweises auf die Patenterteilung:
**01.10.2003 Patentblatt 2003/40**

(21) Anmeldenummer: **96942357.3**

(22) Anmeldetag: **05.12.1996**

(51) Int Cl.[7]: **C07B 55/00**, C07C 233/47, C07C 323/59

(86) Internationale Anmeldenummer:
**PCT/EP96/05438**

(87) Internationale Veröffentlichungsnummer:
**WO 97/021650 (19.06.1997 Gazette 1997/26)**

# (54) VERFAHREN ZUR RACEMISIERUNG VON N-ACETYL-D(L)-ALPHA-AMINOCARBONSÄUREN

PROCESS FOR RACEMIZATION OF N-ACETYL-D(L)-ALPHA-AMINOCARBOXYLIC ACIDS

PROCEDE DE RACEMISATION D'ACIDES N-ACETYL-D(L)-ALPHA-AMINOCARBOXYLIQUES

(84) Benannte Vertragsstaaten:
**AT BE CH DE DK ES FI FR GB IE IT LI NL PT SE**

(30) Priorität: **13.12.1995 DE 19546531**

(43) Veröffentlichungstag der Anmeldung:
**28.10.1998 Patentblatt 1998/44**

(73) Patentinhaber: **Degussa AG**
**40474 Düsseldorf (DE)**

(72) Erfinder:
- **DRAUZ, Karlheinz**
  **D-63579 Freigericht (DE)**
- **BOMMARIUS, Andreas**
  **D-60596 Frankfurt (DE)**
- **KARRENBAUER, Michael**
  **D-78345 Moos (DE)**
- **KNAUP, Günter**
  **D-63486 Bruchköbel (DE)**

(56) Entgegenhaltungen:
**DE-A- 1 964 420**
**DE-A- 2 740 380**
**DE-A- 3 334 849**
**DE-A- 3 435 095**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft ein Verfahren zur Racemisierung von N-Acetyl-D(L)-α-aminocarbonsäuren im nichtwässrigen Zustand durch Erhitzen auf höhere Temperaturen als Raumtemperatur.

N-Acetyl-D(L)-α-aminocarbonsäuren fallen in Form ihrer Salze bei der enzymatischen Hydrolyse der Salze von N-Acetyl-D,L-α-aminocarbonsäuren mittels einer L-Aminosäureacylase an. Sie bestehen überwiegend aus N-Acetyl-D-α-aminocarbonsäuren und können daneben noch geringere Mengen an den entsprechenden N-Acetyl-L-α-aminocarbonsäuren enthalten. Sie werden im allgemeinen nach Abtrennung der bei der Hydrolyse gebildeten L-α-Aminocarbonsäure racemisiert und erneut zur enzymatischen Spaltung eingesetzt.

In der japanischen Offenlegungsschrift JP-A 138603/76 wird offenbart N-Acetyl-D(L)-α-aminocarbonsäuresalze in wäßriger Lösung bei pH 6 - 7 unter Zugabe von mindestens äquimolaren Mengen an Essigsäureanhydrid bei Temperaturen von 40 - 90 °C zu racemisieren.

Bei dieser Racemisierung in Lösung ist besonders nachteilig mit einem nicht unerheblichen Anteil an Nebenprodukten zu rechnen. Nach erfolgter Eindampfung wird das N-Acetylaminosäuresalz durch Zugabe von Mineralsäure in Freiheit gesetzt und die relativ schwer lösliche N-Acetylaminosäure ausgefällt und isoliert, was insgesamt recht aufwendig erscheint.

Aus der GB-A 14 17 060 ist es bereits bekannt, N-Acetyl-D(L)-α-aminocarbonsäuren durch Erhitzen ihrer Schmelze auf höhere Temperaturen zu racemisieren. Zur Erzielung einer vollständigen Racemisierung sind jedoch relativ lange Verweilzeiten erforderlich, was zu einer starken Verfärbung und zur Bildung erheblicher Mengen an Zersetzungsprodukten führt.

Schließlich kennt man aus der DE-A 34 35 095 noch ein weiteres von der Schmelze von N-Acetyl-D(L)-α-aminocarbonsäuren ausgehendes Verfahren, bei welchem man der Schmelze 0,1 bis 2 Gew.-%, bezogen auf die eingesetzte N-Acetyl-D(L)-α-aminocarbonsäure, an Essigsäureanhydrid zusetzt und sie dann für eine Verweilzeit τ (in Minuten) auf eine Temperatur zwischen 115 und 210 °C erhitzt, wobei die Schmelztemperatur der N-Acetyl-D(L)-α-aminocarbonsäure die Untergrenze der Erhitzungstemperatur T (in °C) darstellt und Erhitzungstemperatur und Verweilzeit durch die Beziehung

$$T = In\left( e^{-50\tau + 215} + e^{-\frac{5}{3}\tau + 155} \right)$$

verknüpft sind, und die Schmelze nach Ablauf der Verweilzeit mit einer wäßrigen Alkalimetallhydroxid- oder Ammoniaklösung abschreckt.

Obwohl nach dem in der DE-A 34 35 095 angegebenen Verfahren nur relativ kurze Verweilzeiten für eine vollständige Racemisierung erforderlich sind, die insbesondere beträchtlich unterhalb der Verweilzeiten liegen, die beim Erhitzen der Schmelze in Abwesenheit von Acetanhydrid angewendet werden müssen, und dadurch Verfärbungen und die Bildung von Zersetzungsprodukten verringert werden, kommt es beim Verfahren gemäß diesem Stand der Technik immer noch zu einer merklichen Bildung von Nebenprodukten, vor allem Acetyldipeptiden.

In DE-OS 20 44 680 wird ein Verfahren beschrieben, bei dem das Ammoniumsalz einer optisch aktiven N-Acetyl-α-aminophenylessigsäure in Anwesenheit oder Abwesenheit von Wasser racemisiert wird, indem man es auf über 150 °C für 10 h erhitzt. Da unter diesen Bedingungen Ammoniak aus dem Salz freigesetzt wird, muß die Reaktion in einem geschlossenen Rohr durchgeführt werden.

Angesichts des hierin dargelegten Standes der Technik ist es mithin Aufgabe der Erfindung, ein weiteres Verfahren der eingangs erwähnten Gattung zur Verfügung zu stellen, das die Racemisierung von N-Acetyl-D(L)-α-aminocarbonsäuren in hoher Ausbeute unter möglichst schonenden Bedingungen unter weitgehender Vermeidung der Bildung von Nebenprodukten gestattet.

Gelöst werden diese und weitere nicht näher ausgeführten Probleme durch ein Verfahren zur Racemisierung von N-Acetyl-D(L)-α-aminocarbonsäuren im nicht-wässrigen Zustand durch Erhitzung auf höhere Temperaturen als Raumtemperatur, mit den Merkmalen des kennzeichnenden Teils des Anspruches 1. Vorteilhafte Verfahrensvarianten sind Gegenstand der auf Anspruch 1 rückbezogenen Ansprüche.

Dadurch, daß vor oder während der Temperatureinwirkung wenigstens ein Teil der N-Acetyl-D(L)-α-aminocarbonsäuren in entsprechende N-Acetyl-D(L)-α-aminocarbonsäuresalze überführt wird, wird es vorteilhaft möglich, eine längere Verweilzeit des zu racemisierenden Edukts bei erhöhter Temperatur zu ermöglichen, ohne daß eine Erhöhung des Nebenproduktanteils (insbes. acetylierte Dipeptide) zu befürchten ist.

Gegenüber der aus dem Stand der Technik bekannten Racemisierung der Salze in Lösung wird also die Anwendung einer höheren Temperatur ermöglicht, was zu einer allgemeinen Verbesserung der Racemisierung, insbesondere hinsichtlich Reaktionsgeschwindigkeit und Ausbeute, führt, während die zumindest teilweise Neutralisation des Eduktes vor der Racemisierung überraschenderweise zu einer Verringerung der Nebenproduktbildung führt.

Nachdem die Neutralisation gemäß dem Stand der Technik zeitlich nach der Racemisierung (nach Ablauf der Verweilzeit) durch Abschrecken mit einer wässrigen Alkalimetallhydroxid- oder Ammoniaklösung erreicht wurde, ist die mit der Überführung zumindest eines Teils der N-Acetyl-D(L)-α-aminocarbonsäuren in die entsprechenden Salze verbundene Änderung der Zusammensetzung des zu racemisierenden Gemisches in nicht vorhersehbarer Weise mit verantwortlich für eine günstige Gestaltung des Verhältnisses der Geschwindigkeiten von Racemisierungsreaktion und Nebenproduktbildung.

Insbesondere kann vermutet werden, daß dies auf eine deutlich erhöhte thermische Beständigkeit der Salze im Vergleich zu den freien Acetylaminosäuren zurückzuführen sein könnte.

Im Rahmen der Erfindung wird die Racemisierung der N-Acetyl-D(L)-α-aminocarbonsäuresalze oder eines Gemisches, das diese Salze zusammen mit den entsprechenden freien Säuren aufweist, im "nicht-wässrigen" Zustand durchgeführt. Hierunter wird erfindungsgemäß verstanden, daß die Racemisierung nicht in wässriger Lösung durchzuführen ist. Die absolute Wasserfreiheit ist jedoch nicht Voraussetzung für die erfolgreiche Durchführung des Verfahrens gemäß der Erfindung.

Unter Überführung wenigstens eines Teils der N-Acetyl-D(L)-α-aminocarbonsäuren in die dazugehörigen N-Acetyl-D(L)-α-aminocarbonsäuresalze wird in der Erfindung verstanden, daß bei der Racemisierung selbst ein Gemisch aus freier Säure und Salz oder aber nur das Salz vorliegt.

Sofern ein Gemisch aus Salz und Säure für die Racemisierung verwendet wird, kann die Racemisierungsmischung beispielsweise durch Mischen der entsprechenden reinen Substanzen erhalten werden.

In bevorzugter erfindungsgemäßer Ausführungsform wird jedoch eine wäßrige Lösung der N-Acetyl-D(L)-α-aminocarbonsäure mit einer Base (vorzugsweise NaOH) versetzt. Hierbei wird ein pH-Wert erreicht oder eingestellt, der nicht über 8 liegt.

Vorzugsweise wird eine wäßrige Lösung der N-Acetyl-D(L)-α-aminocarbonsäure oder eine diese im Gemisch mit einem entsprechenden N-Acetyl-D(L)-α-aminocarbonsäuresalz enthaltende wäßrige Lösung auf einen pH-Wert von 2 - 8, bevorzugt 4 - 8, besonders bevorzugt 4,5 - 5,5, gestellt.

Durch die Einstellung auf einen bestimmten pH-Bereich oder -Wert wird vorteilhaft nicht nur die wesentliche Menge der freien Säure in das zugehörige Salz überführt, gleichzeitig wird durch das dabei eingestellte Verhältnis auch ein für die thermische Racemisierung günstiges Verhältnis der interessierenden Reaktionsgeschwindigkeiten erreicht. Der bei dem erfindungsgemäßen Verfahren eingestellte pH-Wert von 2 - 8, vorteilhaft von 4 - 8 und vorzugsweise 4,5 - 5,5, reduziert die Hydrolysegefahr der N-Acetyl-D(L)-α-aminocarbonsäuresalze im Vergleich zu der freien N-Acetylaminosäure erheblich und damit die Nebenproduktbildung (N-Acetyldipeptide), so daß selbst längere Verweilzeiten bzw. höhere Temperaturen nahezu keinen negativen Einfluß auf die Ausbeute haben. Der pH-Wert wird vorzugsweise auf 4,5 - 5,5 eingestellt, weil in diesem pH-Bereich die Pufferwirkung der Mischung am größten ist.

Zur Einstellung des gewünschten pH-Wertes kann grundsätzlich jede dem Fachmann geläufige Base Verwendung finden, die geeignete Salze mit N-Acetyl-D(L)-α-aminocarbonsäuren bilden kann. Bevorzugt werden die Alkali-Laugen. Ganz besonders bevorzugt ist NaOH, weil die pH-Einstellung mit Natronlauge im technischen Maßstab wesentlich erleichtert wird.

Die mit einer Base auf den erfindungsgemäßen pH-Wert-Bereich eingestellte Lösung wird in einer nützlichen Verfahrensmodifikation unter Erhalt eines Rückstandes bis zur Trockne, also zum Erhalt eines Feststoffs, oder zur Schmelze eingedampft.

Dabei ist es von Vorteil, wenn dem erhaltenen Rückstand, sei es als Schmelze oder als trockener Feststoff, zur Racemisierung durch Erhitzen Acetanhydrid zugesetzt wird.

Handelt es sich bei der zu racemisierenden Substanz um eine Schmelze, so ist der Zusatz einer katalytischen Menge an Acetanhydrid bevorzugt, weil dadurch die Geschwindigkeit der Racemisierung deutlich gesteigert werden kann.

Das Aufschmelzen der N-Acetyl-D(L)-α-aminocarbonsäure und das Erhitzen der Schmelze erfolgt vorzugsweise unter Stickstoff. Das Aufschmelzen und das Erhitzen der Schmelze können jedoch auch ohne Schutzgas oder im Vakuum vorgenommen werden.

Obwohl die Verwendung einer Schmelze zur Racemisierung sehr vorteilhaft ist, hat es sich weiterhin unerwartet gezeigt, daß es für die Durchführung des erfindungsgemäßen Verfahrens zur Racemisierung nicht unbedingt erforderlich ist, eine Schmelze der Acetylaminosäuresalze zu erhalten. Im Falle, daß der Schmelzpunkt des Salzes sehr hoch liegt, ist es möglich, den Feststoff mit der erforderlichen Menge Acetanhydrid zu vermischen und diese Mischung auf Temperaturen unter dem Schmelzpunkt der Acetylaminosäuresalze zu erhitzen. Durch das zugesetzte Acetanhydrid kann sich eine partielle Schmelze bilden, die zur Racemisierung ausreichend ist.

Nach diesem Verfahren können z. B. Acetyl-phenylalaninnatrium und Acetyl-valin-natrium bereits bei Temperaturen von 160 °C racemisiert werden.

Die Menge an zuzusetzendem Acetanhydrid ist im Rahmen der Erfindung nicht besonders kritisch. Bevorzugt versetzt man die Schmelze oder partielle Schmelze unter guter Durchmischung mit 2 - 10 Gew.-%, besonders bevorzugt 2 - 6 Gew.-%, Acetanhydrid, bezogen auf die Summe aus N-Acetyl-D(L)-α-aminocarbonsäure und N-Acetyl-D(L)-α-aminocarbonsäuresalz.

Die Temperatur, die zur Racemisierung eingesetzt wird, soll möglichst hoch sein, ohne die Substanzen übermäßig zu schädigen oder Nebenreaktionen in den Vordergrund treten zu lassen.

Ist unter diesen Voraussetzungen der Erhalt einer Schmelze möglich, so wird die Schmelze zur Racemisierung vorteilhaft auf und bei einer Temperatur erhitzt, die 5 bis 10 °C über der Schmelztemperatur der jeweiligen N-Acetyl-D(L)-α-aminocarbonsäure oder des dazugehörigen Salzes liegt, je nachdem, welche Temperatur höher ist.

Ist aufgrund des Schmelzpunktes der beteiligten Substanzen der Erhalt einer vollständigen Schmelze nicht möglich, so ist es günstig, die Temperaturen zur Racemisierung im Bereich von 100 bis 220 °C, vorzugsweise 130 - 180 °C, zu wählen. Diese Bereiche erfüllen in der Regel alle zu stellenden Anforderungen.

Zur Weiterverarbeitung des Racemats kann man jede dem Fachmann hierfür geläufige Verfahrensvariante verwenden. Bevorzugt werden die Schmelze oder partielle Schmelze nach Beendigung des Erhitzens in Wasser aufgenommen und dann der weiteren Verarbeitung zugeführt.

Das erfindungsgemäße Racemisierungsverfahren ist grundsätzlich auf α-Aminocarbonsäuren respektive deren N-acetylierte Derivate anwendbar. Zu den erfolgreich zu racemisierenden Verbindungen gehören u. a. die N-Acetylderivate von Verbindungen der Formel I

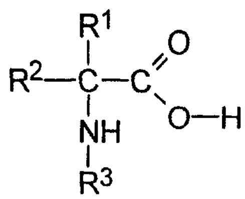

I,

worin

- $R^1$ Wasserstoff oder $C_{1-4}$-Alkyl,
- $R^2$ Wasserstoff, Alkyl, substituiertes Alkyl, Aryl, substituiertes Aryl, Heteroaryl, Arylalkyl, Heteroarylalkyl, Cycloalkyl- oder Cycloalkylalkyl ist, wobei die genannten Reste jeweils wiederum selbst substituiert sein können und/oder Heterpatome enthalten können,
  oder
  $R^1$ und $R^2$ zusammen mit dem sie verbindenden C-Atom einen 3 bis 7-gliedrigen gesättigten Ring bilden,
- $R^3$ Wasserstoff oder $C_{1-4}$-Alkyl ist
  oder
  $R^2$ und $R^3$ zusammen mit dem sie verbindenden N- und C-Atom einen 4 bis 7-gliedrigen gesättigten Ring bilden, der ggf. Heteroatome enthalten kann.

Alkylgruppen können geradkettig oder verzweigt sein, bevorzugt sind Kettenlängen von $C_1$-$C_{12}$ bei geraden Ketten bzw. Kettenlängen von $C_{3-12}$ bei verzweigten Ketten, besonders bevorzugt Kettenlängen von $C_{1-6}$ bei geraden Ketten bzw. $C_{3-6}$ bei verzweigten Ketten, Beispiele sind Methyl, Ethyl, Propyl, Isopropyl, Butyl, Isooctyl, Dodecyl.

Diese Alkylgruppen sind bevorzugt substituiert durch 1 - 3 Amino-, Hydroxyl-, Halogen-, Guanidino-, Harnstoff-, Carboxy-, Carboxamid- und/oder Alkoxycarbonyl-Gruppen.

Arylgruppen können bevorzugt Phenyl- oder substituierte Phenyl-Gruppen sein.

Substituierte Arylgruppen sind bevorzugt Mono-, Di-, oder Trihalogen, Mono-, Di-, oder Trihydroxy, Mono-, Di-, Trialkyl-Phenylgruppen, wobei Halogen Fluor Chlor oder Brom ist, Alkyl $C_{1-4}$-Alkyl, bevorzugt Methyl oder Ethyl.

Heteroaryl-Gruppen sind bevorzugt 5 oder 6-Ring-Systeme mit 1 - 2 Heteroatomen im Ring, die O, N, oder S sein können.

Arylalkyl ist bevorzugt Benzyl. Cycloalkyl und Cycloalkylmethyl sind bevorzugt $C_{3-7}$-Ring-Systeme.

Bevorzugt sind Alanin, Valin, Leucin, Isoleucin, Prolin, Tryptophan, Phenylalanin, Methionin, Serin, Tyrosin, Threonin, Cystein, Asparagin, Glutamin, Histidin, Cystin, Citrullin, Homocystein, Homoserin, Hydroxyprolin, Ornithin, Norvalin sowie Derivate der vorstehend genannten Aminosäuren. Bevorzugt sind N-Acetyl-D(L)-Methionin, -Valin, -Phenylalanin und/oder -Norvalin.

Besonders vorteilhaft wird N-Acetyl-D(L)-Methionin racemisiert. In diesem Falle ist es bevorzugt, eine Schmelze erhältlich durch Eindampfen einer Lösung, die N-Acetyl-D(L)-Methionin aufweist und mit Natronlauge auf einen pH-Wert zwischen 4 und 8 gestellt ist, bei 110 - 180 °C, bevorzugt 150 - 160 °C, unter guter Durchmischung mit mindestens 2 und maximal 6 Gew.-% Essigsäureanhydrid zu versetzen und nach einer Reaktionszeit von 2 - 30 min, vorzugsweise 10 - 15 min, in Wasser aufzunehmen.

Das erfindungsgemäße Verfahren ist grundsätzlich zur Racemisierung von N-Acetyl-D(L)-α-aminocarbonsäure geeignet, gleichgültig welchen Ursprungs. Die Racemisierung läßt sich jedoch hervorragend in einen Prozeß zur Gewinnung optisch aktiver α-Aminosäuren integrieren.

Dabei kennzeichnet sich solch ein integriertes Verfahren beispielsweise zur Gewinnung von L-Methionin dadurch, daß die N-Acetyl-D(L)-Methionin aufweisende Lösung eine Mutterlauge und die in Wasser aufgenommene racemisierte Schmelze eine Rückführlösung ist, die in einem Verfahren zur Gewinnung von optisch aktivem L-Methionin verwendet werden, bei dem D,L-Methionin zunächst mit Essigsäure/Acetanhydrid N-acetyliert wird, das N-Acetyl-D, L-Methionin-Racemat enzymatisch gespalten wird, das L-Methionin unter Rückbehalt einer Mutterlauge abgetrennt wird und das nicht umgesetzte N-Acetyl-D(L)-Methionin der Mutterlauge nach Racemisierung als Rückführlösung zur enzymatischen Racematspaltung rezykliert wird.

Das bei dem erfindungsgemäßen Verfahren eingesetzte N-Acetyl-D(L)-Methioninnatriumsalz und N-Acetyl-D(L)-Methionin kann dabei zweckmäßigerweise so gewonnen werden, daß man die bei der enzymatischen Hydrolyse nach erfolgter Isolierung des L-Methionins anfallende Mutterlauge über einen stark sauren Kationenaustauscher schickt, der die enthaltenen Kationen und die restliche L-α-Aminocarbonsäure adsorbiert. Die aus dem Ionenaustauscher austretende Lösung besteht dann praktisch nur noch aus Wasser, Essigsäure und N-Acetyl-D(L)-Methionin. Sie wird beispielsweise unter schonenden Bedingungen bei niedrigen Temperaturen im Vakuum, beispielsweise in einer Kombination aus Fallfilmverdampfer und Dünnschichtverdampfer mit Feststoffaustrag zwecks Entfernung der die enzymatische Umsetzung inhibierenden Essigsäure eingedampft und in wäßrige Natronlauge bei pH 4 - 8, vorzugsweise 4,5 - 5,5, eingetragen. Alternativ dazu kann die NaOH auch bereits vor Entfernung der Essigsäure zugesetzt werden. Dadurch wird der pH-Wert schon vor der Eindampfung erhöht, wodurch die Hydrolyse, im gewählten Beispiel von N-Ac-D(L)-Methionin, reduziert werden kann.

Auch die zum Aufschmelzen der N-Acetyl-D(L)-α-aminocarbonsäure erforderliche Verweilzeit soll zweckmäßigerweise möglichst kurz gehalten werden. Nimmt man das Aufschmelzen in einem beheizten Extruder vor, so reicht für das vollständige Aufschmelzen im allgemeinen eine Verweilzeit von weniger als einer Minute. In diesem Fall kann der Extruder die Schmelze in ein beheiztes Reaktionsrohr fördern, wo zu Beginn der Verweilstrecke eine entsprechend ausgelegte Pumpe kontinuierlich die erforderliche Menge an Essigsäureanhydrid über ein Mischsystem zudosiert.

Die Erfindung wird durch die nachfolgenden Beispiele näher erläutert. Alle Prozentangaben bedeuten, wenn nicht anders angegeben, Gewichtsprozente.

Die eingesetzten N-Acetyl-D(L)-α-aminocarbonsäuren und die racemisierten Proben werden jeweils auf ihren spezifischen Drehwert $[\alpha]_D^{20}$ in Grad $cm^3/dm \cdot g$ untersucht.

Beispiel 1: Racemisierung von N-Acetyl-D(L)-methioninnatrium in der Schmelze

Versuch 1

10 g (0.053 mol) N-Acetyl-D(L)-methionin wurden mit ca. 25 ml 8-gew.-%iger Natronlauge auf einen pH-Wert von 5 gestellt und im Vakuum bei 5 kPa (50 mbar) bis zur Schmelze eingedampft. Anschließend wurden unter Rühren 0,2 g Essigsäureanhydrid bei einer Temperatur von 155 °C zudosiert. Diese Schmelze wurde dann für die Dauer von 30 Minuten bei 155 °C gehalten und anschließend in Wasser aufgenommen.

$[\alpha]_D^{20}$ vor der Racemisierung: + 21,3° (c = 4 in Wasser)
$[\alpha]_D^{20}$ nach der Racemisierung: ± 0° (c = 4 in Wasser)

Gehalt an Dipeptiden nach erfolgter Racemisierung:

0,3 Gew.-% bezogen auf N-Acetylmethionin.

Versuch 2

Beispiel 1 wurde wiederholt mit dem Unterschied, daß man durch Zugabe von ca. 20 ml 8 gew.-%iger Natronlauge einen pH-Wert von 4 einstellte.

$[\alpha]_D^{20}$ nach der Racemisierung: + 0,2° (c = 4 in $H_2O$)

Gehalt an Dipeptiden nach erfolgter Racemisierung:

0,8 Gew.-% bezogen auf N-Acetylmethionin.

Versuch 3

Beispiel 1 wurde wiederholt mit dem Unterschied, daß man durch Zugabe von ca. 26 ml 8 gew.-%iger Natronlauge einen pH-Wert von 6 einstellte.

$[\alpha]_D^{20}$ nach der Racemisierung: ± 0° (c = 4 in Wasser)

Gehalt an Dipeptiden nach erfolgter Racemisierung:

0,2 Gew.-% bezogen auf N-Acetylmethionin.

Versuch 4

Beispiel 1 wurde wiederholt mit dem Unterschied, daß man die Schmelze bei einer Temperatur von 110 - 120 °C einstellte und 0,3 g Essigsäureanhydrid zudosierte.

$[\alpha]_D^{20}$ nach Racemisierung: + 0,5° (c = 4 in Wasser)

Gehalt an Dipeptiden nach erfolgter Racemisierung:

0,05 Gew.-% bezogen auf N-Acetylmethionin.

Versuch 5

Beispiel 1 wurde wiederholt mit dem Unterschied, daß man die Schmelztemperatur auf 180 °C erhöhte und die Verweilzeit auf 10 Minuten einstellte.

$[\alpha]_D^{20}$ nach Racemisierung: 0° (c = 4 in Wasser)

Gehalt an Dipeptiden nach Racemisierung:

0,1 Gew.-% bezogen auf N-Acetylmethionin.

Versuch 6

Beispiel 5 wurde wiederholt mit dem Unterschied, daß man die Verweilzeit auf 30 Minuten erhöhte.

$[\alpha]_D^{20}$ nach der Racemisierung: ± 0° (c = 4 in Wasser)

Gehalt an Dipeptiden nach Racemisierung:

0,8 Gew.-% bezogen auf N-Acetylmethionin.

Versuch 7

Beispiel 1 wurde wiederholt mit dem Unterschied, daß man durch Zugabe von ca. 27 ml 8 gew.-%iger Natronlauge auf pH 8 stellte und 0,3 g Essigsäureanhydrid zudosierte.

$[\alpha]_D^{20}$ nach der Racemisierung: ± 0° (c = 4 in Wasser)

Gehalt an Dipeptiden nach Racemisierung:

0,4 Gew.-% bezogen auf N-Acetylmethionin.

Versuch 8

Beispiel 1 wurde wiederholt mit dem Unterschied, daß man 0,6 g Essigsäureanhydrid zudosierte.

$[\alpha]_D^{20}$ nach der Racemisierung: ± 0° (c = 4 in Wasser)

Gehalt an Dipeptiden nach Racemisierung:

0,5 Gew.-% bezogen auf N-Acetylmethionin.

Vergleichsversuch 9

Versuch 1 wurde wiederholt ohne Zugabe von Natronlauge (pH ca. 1,6), d. h. freies N-Acetyl-D(L)-Methionin wurde thermisch racemisiert, indem man die Verweilzeit der Schmelze nach erfolgter Zudosierung des Essigsäureanhydrids bei 155 °C auf 30 Minuten einstellt.

$[\alpha]_D^{20}$ nach der Racemisierung: ± 0° (c = 4 in Wasser)

Gehalt an Dipeptiden nach Racemisierung:

5,5 Gew.-% bezogen auf N-Acetylmethionylmethionin.

Die Ergebnisse dieser Versuche sind in der folgenden Tabelle zusammengefaßt:

| **Versuch/ Vgl. -Vers.** | **pH-Wert** | **$Ac_2O$** | **Temp.** | **Verweil-zeit** | **$[\alpha]_D^{20}$** | **Ac-Met-Met** |
|---|---|---|---|---|---|---|
| **1** | 5 | 2 % | 155 °C | 30 min | 0 | 0.3 % |
| **2** | 4 | 2 % | 155 °C | 30 min | +0.2 | 0.8 % |
| **3** | 6 | 2 % | 155 °C | 30 min | 0 | 0.2 % |
| **4** | 5 | 3 % | 110-20 °C | 30 min | +0.5 | 0.05 % |
| **5** | 5 | 2 % | 180 °C | 10 min | 0 | 0.1 % |
| **6** | 5 | 2 % | 180 °C | 30 min | 0 | 0.8 % |
| **7** | 8 | 3 % | 155 °C | 30 min | 0 | 0.4 % |
| **8** | 5 | 6 % | 155 °C | 30 min | 0 | 0.5 % |
| **9** | 1.6 | 2 % | 155 °C | 30 min | 0 | 5.5 % |

Beispiel 2: Racemisierung von N-Acetyl-L-phenylalaninnatriumsalz

Bei 150 °C im Vakuum getrocknete Proben von N-Acetyl-Lphenylalanin-natriumsalzen wurden mit Acetanhydrid vermischt und erwärmt. Zur Verfolgung der Racemisierung wurde der Drehwert ($[\alpha]_D^{20}$, c = 1 in 1 N HCl) bestimmt. Vor der Racemisierung betrug dieser Drehwert + 21.6. Die Ergebnisse sind in der nachfolgenden Tabelle zusammengefaßt:

| **$Ac_2O$** | **Temp.** | **Verweilzeit** | **$[\alpha]_D^{20}$** |
|---|---|---|---|
| 2 % | 140 °C | 15 min | 9.0 |
| 2 % | 160 °C | 15 min | 2.9 |
| 2 % | 180 °C | 15 min | 1.9 |
| 5 % | 160 °C | 15 min | 0 |
| - | 230 °C | 15 min | 10.8 |

Beispiel 3: Racemisierung von N-Acetyl-L-valin-natriumsalz

Bei 150 °C im Vakuum getrocknete Proben von N-Acetyl-L-valin-natriumsalzen wurden mit Acetanhydrid vermischt und erwärmt. Zur Verfolgung der Racemisierung wurde der Drehwert ($[\alpha]_D^{20}$, c = 1 in 1 N HCl) bestimmt. Vor der Racemisierung betrug dieser Drehwert + 11.9. Die Ergebnisse sind in der folgenden Tabelle zusammengefaßt:

| **$Ac_2O$** | **Temp.** | **Verweilzeit** | **$[\alpha]_D^{20}$** |
|---|---|---|---|
| 2 % | 140 °C | 15 min | 10.2 |
| 2 % | 160 °C | 15 min | 6.5 |
| 2 % | 180 °C | 15 min | 4.8 |
| 5 % | 160 °C | 15 min | 1.6 |
| 5 % | 230 °C | 15 min | 0 |
| - | 230 °C | 15 min | 8.3 |

Beispiel 4: Recyclierung einer N-Acetyl-D(L)-Methioninnatriumsalz-Mutterlauge

717 g (3.75 mol) N-Acetyl-D,L-methionin und 140 g (3.50 mol) Natriumhydroxid wurden in Wasser gelöst und auf 1.5 1 aufgefüllt. Der pH-Wert der Lösung wurde mit 50%-iger Natronlauge auf 7 gestellt. Nach Zugabe von 3.6 g Acylase (Aktivität 31.000 E/g) wurde 5 Tage bei Raumtemperatur gerührt. Die Suspension wurde auf 5 °C abgekühlt, der Niederschlag abfiltriert, mit 300 ml Eiswasser gewaschen und getrocknet. Man erhielt 174 g (1.17 mol) L-Methionin. Das Filtrat (1800 g), das neben 21.8 Gew.-% N-Acetyl-D(L)-methionin 2.8 Gew.-% L-Methionin enthielt, wurde auf 60 °C erwärmt und mit 60 g (0.60 mol) Acetanhydrid versetzt. Nach 30 min war in der Lösung kein Methionin nachweisbar. Es wurden 223 g (1.17 mol) N-Acetyl-D,L-methionin zugegeben und im Vakuum auf 1200 g eingeengt. Diese Lösung wurde mit einem Sambay-Verdampfer bei 170 °C und 1 kPa (10 mbar) auf 752 g eingeengt. In die noch warme Schmelze wurden 15 g Acetanhydrid eingerührt, 10 min auf 140 °C erhitzt und dann in Wasser gelöst und auf 1.5 1 aufgefüllt. Der Drehwert der Lösung war Null. Der Gehalt an N-Acetyldipeptid betrug 0.8 % bez. auf N-Acetyl-methionin. Nachdem der pH-Wert mit Natronlauge auf 7 gestellt wurde, wurde diese Lösung wie oben beschrieben erneut zur Acylasespaltung eingesetzt. Man erhielt 166 g (1.11 mol) L-Methionin.

Beispiel 5: Recyclierung einer N-Acetyl-D(L)-Norvalinnatriumsalz-Mutterlauge

597 g (3.75 mol) N-Acetyl-D,L-norvalin und 140 g (3.50 mol) Natriumhydroxid wurden in Wasser gelöst und auf 1.5 1 aufgefüllt. Der pH-Wert der Lösung wurde mit 50%-iger Natronlauge auf 7 gestellt. Nach Zugabe von 3.0 g Acylase (Aktivität 31.000 E/g) wurde 5 Tage bei Raumtemperatur gerührt. Die Suspension wurde auf 5 °C abgekühlt, der Niederschlag abfiltriert, mit 300 ml Eiswasser gewaschen und getrocknet. Man erhielt 157 g (1.34 mol) L-Norvalin. Das Filtrat (1393 g), das neben 22.5 Gew.-% N-Acetyl-D(L)-norvalin 3.1 Gew.-% L-Norvalin enthielt, wurde auf 60 °C erwärmt und mit 64 g (0.62 mol) Acetanhydrid versetzt. Nach 30 min war in der Lösung kein Norvalin nachweisbar. Es wurden 213 g (1.34 mol) N-Acetyl-D,L-norvalin zugegeben und im Vakuum auf 950 g eingeengt. Diese Lösung wurde mit einem Sambay-Verdampfer bei 170 °C und 1 kPa (10 mbar) auf 644 g eingeengt. In die noch warme Schmelze wurden 13 g Acetanhydrid eingerührt, 10 min auf 140 °C erhitzt und dann in Wasser gelöst und auf 1.5 1 aufgefüllt. Eine chromatographische ee-Bestimmung erbrachte ein Verhältnis D/L von 51.3 % zu 48.7 %. Der Gehalt an N-Acetyldipeptid betrug 1.2 % bez. auf N-Acetyl-norvalin. Nachdem der pH-Wert mit Natronlauge auf 7 gestellt wurde, wurde diese Lösung wie oben beschrieben erneut zur Acylasespaltung eingesetzt. Man erhielt 113 g (0.97 mol) L-Norvalin.

**Patentansprüche**

1. Verfahren zur Racemisierung von N-Acetyl-D(L)-α-aminocarbonsäuren im nicht-wässrigen Zustand durch Erhitzen auf höhere Temperaturen als Raumtemperatur,
**dadurch gekennzeichnet,**
**daß** vor oder während der Temperatureinwirkung wenigstens ein Teil der N-Acetyl-D(L)-α-aminocarbonsäuren in ihre entsprechenden Alkali- oder Erdalkalisalze überführt und unter Zusatz von Acetanhydrid erhitzt wird.

2. Verfahren nach Anspruch 1,
**dadurch gekennzeichnet,**
**daß** eine feste Mischung oder eine Schmelze eines Feststoffgemisches aus N-Acetyl-D(L)-α-aminocarbonsäure und einem entsprechenden N-Acetyl-D(L)-α-aminocarbonsäuresalz durch Temperatureinwirkung racemisiert wird.

3. Verfahren nach Anspruch 1,
**dadurch gekennzeichnet,**
**daß** eine wäßrige Lösung der N-Acetyl-D(L)-α-aminocarbonsäure oder eine diese im Gemisch mit einem entsprechenden N-Acetyl-D(L)-α-aminocarbonsäuresalz enthaltende wäßrige Lösung mit einer Base auf einen pH-Wert von 2 - 8, bevorzugt 4 - 8, besonders bevorzugt 4,5 - 5,5, gestellt wird und die so eingestellte Lösung unter Erhalt eines Rückstandes bis zum trocknen Feststoff oder zur Schmelze eingedampft wird.

4. Verfahren nach Anspruch 3,
**dadurch gekennzeichnet,**
**daß** zur Einstellung des pH-Wertes NaOH verwendet wird.

5. Verfahren nach Anspruch 3 oder 4,
**dadurch gekennzeichnet,**
**daß** eine Mischung eines trockenen Feststoffs mit Acetanhydrid unter Ausbildung einer partiellen Schmelze erhitzt

wird.

**6.** Verfahren nach Anspruch 3 und 4 oder 5,
**dadurch gekennzeichnet,**
**daß** man die Schmelze oder partielle Schmelze unter guter Durchmischung mit 2 - 10 Gew.-%, bevorzugt 2 - 6 Gew.-%, Acetanhydrid, bezogen auf N-Acetyl-D(L)-α-aminocarbonsäure und N-Acetyl-D(L)-α-aminocarbonsäuresalz, versetzt.

**7.** Verfahren nach Anspruch 3 und 4 oder Anspruch 6, soweit dieser auf Anspruch 3 und 4 rückbezogen ist,
**dadurch gekennzeichnet,**
**daß** die Schmelze zur Racemisierung bei einer Temperatur erhitzt wird, die 5 bis 10 °C über der Schmelztemperatur der jeweiligen N-Acetyl-D(L)-α-aminocarbonsäure oder des dazugehörigen Salzes liegt, je nachdem, welche Temperatur höher ist.

**8.** Verfahren nach einem oder mehreren der Ansprüche 3 bis 7,
**dadurch gekennzeichnet,**
**daß** die Schmelze oder partielle Schmelze nach Beendigung des Erhitzens in Wasser aufgenommen wird.

**9.** Verfahren nach einem der vorherigen Ansprüche,
**dadurch gekennzeichnet,**
**daß** N-Acetyl-D(L)-Methionin, -Valin, -Phenylalanin und/oder -Norvalin racemisiert werden.

**10.** Verfahren nach Anspruch 9,
**dadurch gekennzeichnet,**
**daß** N-Acetyl-D(L)-Methionin racemisiert wird.

**11.** Verfahren nach Anspruch 10,
**dadurch gekennzeichnet,**
**daß** eine Schmelze erhältlich durch Eindampfen einer Lösung, die N-Acetyl-D(L)-Methionin aufweist und mit Natronlauge auf einen pH-Wert zwischen 4 und 8 gestellt ist, bei 110 - 180 °C, bevorzugt 150 - 160 °C, unter guter Durchmischung mit mindestens 2 und maximal 6 Gew.-% Essigsäureanhydrid versetzt und nach einer Reaktionszeit von 2 - 30 min, vorzugsweise 10 - 15 min, in Wasser aufgenommen wird.

**12.** Verfahren nach Anspruch 11,
**dadurch gekennzeichnet,**
**daß** die N-Acetyl-D(L)-Methionin aufweisende Lösung eine Mutterlauge und die in Wasser aufgenommene racemisierte Schmelze eine Rückführlösung ist, die in einem Verfahren zur Gewinnung von optisch aktivem L-Methionin verwendet werden,
bei dem D,L-Methionin zunächst mit Essigsäure/Acetanhydrid N-acetyliert wird, das N-Acetyl-D,L-Methionin-Racemat enzymatisch gespalten wird, das L-Methionin unter Rückbehalt einer Mutterlauge abgetrennt wird und das nicht umgesetzte N-Acetyl-D(L)-Methionin der Mutterlauge nach Racemisierung als Rückführlösung zur enzymatischen Racemat-Spaltung rezykliert wird.

## Claims

**1.** Process for the racemisation of N-acetyl-D(L)-α-aminocarboxylic acids in the non-aqueous state by heating them to temperatures above room temperature,
**characterised in that**
before or during the application of heat at least a part of the N-acetyl-D(L)-α-aminocarboxylic acids is converted into their corresponding alkali or alkaline-earth salts and heated with addition of acetic anhydride.

**2.** Process according to claim 1,
**characterised in that**
a solid mixture or a melt of a solids mixture comprising N-acetyl-D(L)-α-aminocarboxylic acid and a corresponding N-acetyl-D(L)-α-aminocarboxylic acid salt is racemised by the application of heat.

**3.** Process according to claim 1,

**characterised in that**
an aqueous solution of N-acetyl-D(L)-α-aminocarboxylic acid or an aqueous solution containing said acid mixed with a corresponding N-acetyl-D(L)-α-aminocarboxylic acid salt is adjusted with a base to a pH of 2 to 8, preferably 4 to 8, particularly preferably 4.5 to 5.5, and the solution adjusted in this way is evaporated to the dry solid or to the melt with formation of a residue.

**4.** Process according to claim 3,
**characterised in that**
NaOH is used to adjust the pH.

**5.** Process according to claim 3 or 4,
**characterised in that**
a mixture of a dry solid with acetic anhydride is heated with formation of a partial melt.

**6.** Process according to claim 3 and 4 or 5,
**characterised in that**
2 to 10 wt.%, preferably 2 to 6 wt.%, of acetic anhydride, relative to N-acetyl-D(L)-α-aminocarboxylic acid and N-acetyl-D(L)-α-aminocarboxylic acid salt, are added to the melt or partial melt and mixed together thoroughly.

**7.** Process according to claim 3 and 4 or claim 6, provided that the latter refers back to claim 3 and 4,
**characterised in that**
the melt for racemisation is heated at a temperature that is 5 to 10 °C above the melting point of the particular N-acetyl-D(L)-α-aminocarboxylic acid or of the associated salt, depending on which temperature is higher.

**8.** Process according to one or more of claims 3 to 7,
**characterised in that**
the melt or partial melt is taken up in water following the end of the heating process.

**9.** Process according to one of the preceding claims,
**characterised in that**
N-acetyl-D(L)-methionine, -valine, -phenylalanine and/or -norvaline are racemised.

**10.** Process according to claim 9,
**characterised in that**
N-acetyl-D(L)-methionine is racemised.

**11.** Process according to claim 10,
**characterised in that**
at least 2 and at most 6 wt.% of acetic anhydride are added with thorough mixing to a melt obtainable by evaporating a solution displaying N-acetyl-D(L)-methionine and adjusted with sodium hydroxide solution to a pH of between 4 and 8, at 110 to 180 °C, preferably 150 to 160 °C, and after a reaction time of 2 to 30 min, preferably 10 to 15 min, it is taken up in water.

**12.** Process according to claim 11,
**characterised in that**
the solution displaying N-acetyl-D(L)-methionine is a mother liquor and the racemised melt taken up in water is a return solution, which is used in a process to obtain optically active L-methionine,
wherein D,L-methionine is first N-acetylated with acetic acid/acetic anhydride, the N-acetyl-D,L-methionine racemate is split enzymatically, the L-methionine is separated off with retention of a mother liquor and after racemisation the unreacted N-acetyl-D(L)-methionine from the mother liquor is recycled as a return solution for enzymatic racemate splitting.

**Revendications**

**1.** Procédé pour la racémisation d'acides N-acétyl-D(L)-α-aminocarboxyliques dans un état non aqueux par chauffage à des températures supérieures à la température ambiante, **caractérisé en ce qu'**au moins une partie des acides N-acétyl-D(L)-α-aminocarboxyliques est transformée en leurs sels alcalins ou alcalino-terreux correspondants, avant ou pendant l'action de la température, et est chauffée avec addition d'anhydride acétique.

**2.** Procédé selon la revendication 1, **caractérisé en ce qu'**un mélange solide ou une masse fondue d'un mélange de solides d'acide N-acétyl-D(L)-α-aminocarboxylique et d'un sel d'acide N-acétyl-D(L)-α-aminocarboxylique correspondant est racémisé par action de la température.

**3.** Procédé selon la revendication 1, **caractérisé en ce qu'**une solution aqueuse de l'acide N-acétyl-D(L)-α-aminocarboxylique ou une solution aqueuse contenant celui-ci en mélange avec un sel d'acide N-acétyl-D(L)-α-aminocarboxylique correspondant est réglée à un pH de 2 à 8, de préférence de 4 à 8, de manière particulièrement préférée de 4,5 à 5,5 et la solution ainsi réglée est évaporée jusqu'au solide sec ou une masse fondue avec obtention d'un résidu.

**4.** Procédé selon la revendication 3, **caractérisé en ce qu'**on utilise du NaOH pour le réglage du pH.

**5.** Procédé selon la revendication 3 ou 4, **caractérisé en ce qu'**on chauffe un mélange d'un solide sec avec de l'anhydride acétique en formant une masse fondue partielle.

**6.** Procédé selon la revendication 3 et 4 ou 5, **caractérisé en ce qu'**on additionne la masse fondue ou la masse fondue partielle, sous agitation intense, de 2 à 10% en poids, de préférence de 2 à 6% en poids, d'anhydride acétique par rapport à l'acide N-acétyl-D(L)-α-aminocarboxylique et le sel de l'acide N-acétyl-D(L)-α-aminocarboxylique.

**7.** Procédé selon la revendication 3 et 4 ou la revendication 6, pour autant que celle-ci se réfère à la revendication 3 et 4, **caractérisé en ce que** la masse fondue est chauffée, pour la racémisation, à une température supérieure de 5 à 10°C à la température de fusion de l'acide N-acétyl-D(L)-α-aminocarboxylique ou du sel correspondant, en fonction de la température la plus élevée.

**8.** Procédé selon l'une ou plusieurs des revendications 3 à 7, **caractérisé en ce que** la masse fondue ou la masse fondue partielle est reprise dans l'eau après la fin du chauffage.

**9.** Procédé selon l'une quelconque des revendications précédentes, **caractérisé en ce qu'**on racémise de la N-acétyl-D(L)-méthionine, de la N-acétyl-D(L)-valine, de la N-acétyl-D(L)-phénylalanine et/ou de la N-acétyl-D(L)-norvaline.

**10.** Procédé selon la revendication 9, **caractérisé en ce qu'**on racémise de la N-acétyl-D(L)-méthionine.

**11.** Procédé selon la revendication 10, **caractérisé en ce qu'**une masse fondue, pouvant être obtenue par concentration par évaporation d'une solution qui présente de la de la N-acétyl-D(L)-méthionine et qui a été réglée à un pH entre 4 et 8 avec de la lessive de soude caustique, est additionnée, à 110-180°C, de préférence à 150-160°C, sous agitation intense, d'au moins 2 et d'au plus 6% en poids d'anhydride de l'acide acétique et est reprise dans l'eau après un temps de réaction de 2 à 30 minutes, de préférence de 10 à 15 minutes.

**12.** Procédé selon la revendication 11, **caractérisé en ce que** la solution présentant la N-acétyl-D(L)-méthionine est une lessive-mère et la masse fondue racémisée reprise dans l'eau est une solution de recyclage, qui sont utilisées dans un procédé de préparation de L-méthionine optiquement active, dans lequel la D,L-méthionine est d'abord N-acétylée avec un mélange acide acétique/anhydride acétique, le racémate N-acétyl-D,L-méthionine est dissocié enzymatiquement, la L-méthionine est séparée en retenant une lessive-mère et la N-acétyl-D(L)-méthionine non transformée de la lessive-mère est recyclée après la racémisation comme solution de recyclage dans la dissociation enzymatique du racémate.